(19) Europäisches Patentamt
European Patent Office
Office européen des brevets

(11) Veröffentlichungsnummer : **0 112 531**
**B1**

(12) **EUROPÄISCHE PATENTSCHRIFT**

(45) Veröffentlichungstag der Patentschrift : **04.03.87**

(21) Anmeldenummer : **83112467.2**

(22) Anmeldetag : **12.12.83**

(51) Int. Cl.⁴ : **C 07 C 69/712**, C 07 D213/55, C 07 C 67/14, C 07 C 67/31, A 01 N 43/40, A 01 N 37/02, C 07 C143/68, C 07 C139/00, C 07 C149/20, C 07 C147/02, C 07 C147/14

(54) **Optisch aktive Phenoxypropionsäure-Derivate.**

(30) Priorität : **24.12.82 DE 3247930**

(43) Veröffentlichungstag der Anmeldung : **04.07.84 Patentblatt 84/27**

(45) Bekanntmachung des Hinweises auf die Patenterteilung : **04.03.87 Patentblatt 87/10**

(84) Benannte Vertragsstaaten : **AT BE CH DE FR GB IT LI NL**

(56) Entgegenhaltungen :
**EP-A- 0 068 260**
**EP-A- 0 092 112**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Moriya, Koichi**
**Namiki-cho 39-15**
**Hachioji-shi Tokyo (JP)**
Erfinder : **Priesnitz, Uwe, Dr.**
**Severinstrasse 58**
**D-5650 Solingen 1 (DE)**
Erfinder : **Riebel, Hans-Jochem, Dr.**
**In der Beek 92**
**D-5600 Wuppertal 1 (DE)**
Erfinder : **Eue, Ludwig, Dr.**
**Paul-Klee-Strasse 36**
**D-5090 Leverkusen 1 (DE)**
Erfinder : **Schmidt, Robert R., Dr.**
**Im Waldwinkel 110**
**D-5060 Bergisch-Gladbach 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 112 531 B1

**Beschreibung**

Die Erfindung betrifft neue rechtsdrehende Enantiomere von Phenoxypropionsäure-Derivaten, mehrere Verfahren zu deren Herstellung und deren Verwendung als Herbizide. Unter rechtsdrehenden Enantiomeren sind hier jeweils diejenigen optisch aktiven Verbindungen zu verstehen, welche die Schwingungsebene von linear polarisiertem Licht nach rechts drehen.

Es ist bereits bekannt, daß zahlreiche Phenoxypropionsäure-Derivate herbizide Eigenschaften besitzen (vgl. DE-OS 2 617 804 und US-PS 4 046 553). So können zum Beispiel die Racemate des 2-[4-(3,5-Dichlor-pyridyl-2-oxy)-phenoxy]-propionsäure-benzylesters und des 2-[4-(4-Trifluormethyl-phenoxy)-phenoxy]-propionsäure-(2-phenoxy)-ethylesters zur Bekämpfung von Unkraut eingesetzt werden. Die Wirkung dieser Stoffe ist jedoch nicht immer ausreichend.

Weiterhin werden Racemate von erfindungsgemäßen optisch aktiven Phenoxypropionsäure-Derivaten in den nicht vorveröffentlichten Patentanmeldungen EP-A-68 260 und EP-A-92 112 beschrieben.

Es wurden jetzt die neuen rechtsdrehenden Enantiomeren der Phenoxypropionsäure-Derivate der Formel

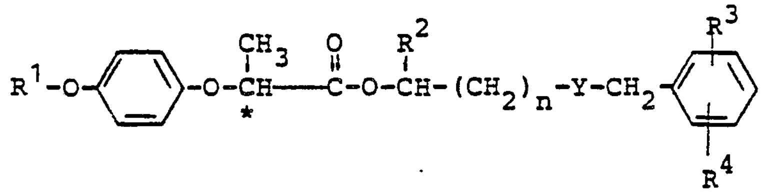

(I)

in welcher $R^1$ für die Reste der Formeln

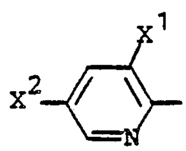

oder

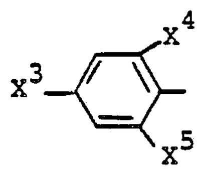

steht, worin

$X^1$ für Wasserstoff oder Halogen steht,
$X^2$ für Halogen oder Trifluormethyl steht,
$X^3$ für Halogen oder Trifluormethyl steht,
$X^4$ für Wasserstoff oder Halogen steht und
$X^5$ für Wasserstoff oder Halogen steht,
Y für Sauerstoff oder den Rest $SO_m$ steht, wobei
m für 0, 1 oder 2 steht,
$R^2$ für Wasserstoff oder Methyl steht,
n für 1 oder 2 steht und
$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,

gefunden.

Weiterhin wurde gefunden, daß man die neuen rechtsdrehenden Enantiomeren der Phenoxypropionsäure-Derivate der Formel (I) erhält, wenn man

a) rechtsdrehende Enantiomere von Phenoxypropionsäurechloriden der Formel

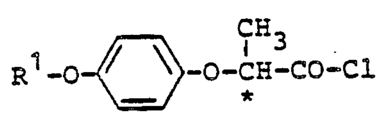

(II)

in welcher $R^1$ die oben angegebene Bedeutung hat, mit Hydroxyverbindungen der Formel

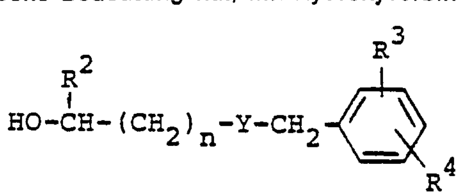

(III).

in welcher $R^2$, $R^3$, $R^4$, Y und n die oben angegebene Bedeutung haben, gegebenenfalls in Gegenwart eines Säureakzeptors sowie gegebenenfalls in Gegenwart eines Verdünnungsmittels umsetzt, und gegebenenfalls anschließend oxidiert,

oder

b) Phenol-Derivate der Formel

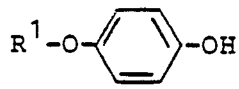

(IV)

in welcher $R^1$ die oben angegebene Bedeutung hat, mit linksdrehenden Enantiomeren von Propionsäure-Derivaten der Formel

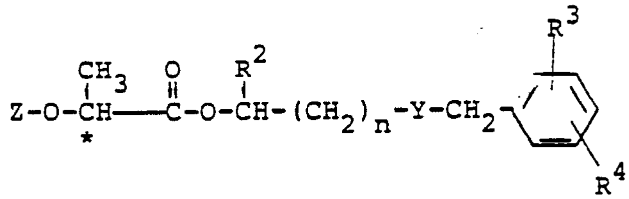

(V)

in welcher

$R^2$, $R^3$, $R^4$, Y und n die oben angegebene Bedeutung haben und

Z für Tosyl oder Mesyl steht,

gegebenenfalls in Gegenwart eines Säureakzeptors sowie gegebenenfalls in Gegenwart eines Verdünnungsmittels umsetzt und gegebenenfalls anschließend oxidiert.

Schließlich wurde gefunden, daß sich die neuen rechtsdrehenden Enantiomeren der Phenoxypropionsäure-Derivate der Formel (I) durch eine hervorragende herbizide Wirksamkeit auszeichnen.

Überraschenderweise besitzen die erfindungsgemäßen rechtsdrehenden Enantiomeren der Phenoxypropionsäure-Derivate der Formel (I) wesentlich bessere herbizide Eigenschaften als die aus dem Stand der Technik bekannten Racemate des 2-[4-(3,5-Dichlor-pyridyl-2-oxy)-phenoxy]-propionsäurebenzylesters und des 2-[4-(4-Trifluormethyl-phenoxy)-phenoxy]-propionsäure-(2-phenoxy)-ethylesters, welches konstitionell ähnliche Stoffe analoger Wirkungsart sind.

Die erfindungsgemäßen Verbindungen sind durch die Formel (I) allgemein definiert. In diser Formel, in der das asymmetrische Kohlenstoffatom durch ein (*) gekennzeichnet ist, steht $R^1$ für die Reste der Formeln

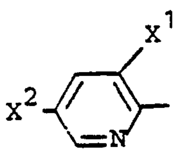

und

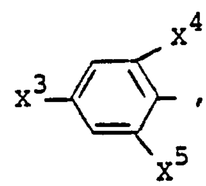

,

in welchen

$X^1$ für Wasserstoff oder Chlor steht,

$X^2$ für Chlor oder Trifluormethyl steht,

$X^3$ für Chlor oder Trifluormethyl steht,

$X^4$ für Wasserstoff oder Chlor steht und

$X^5$ für Wasserstoff oder Chlor steht.

Y steht für Sauerstoff oder den Rest $SO_m$, wobei m für 0, 1 oder 2 steht, und $R^2$ steht für Wasserstoff oder Methyl. Der Index n steht für 1 oder 2 und die Reste $R^3$ und $R^4$ stehen unabhängig voneinander vorzugsweise für Wasserstoff, Fluor, Chlor, Brom, Iod, Alkyl mit 1 bis 2 Kohlenstoffatomen, Alkoxy mit 1 oder 2 Kohlenstoffatomen, Alkylthio mit 1 oder 2 Kohlenstoffatomen, Nitro, Cyano oder Alkoxycarbonyl mit 1 oder 2 Kohlenstoffatomen in der Alkoxygruppe.

Besonders bevorzugt sind diejenigen rechtsdrehenden Enantiomeren der Phenoxypropionsäure-Derivate der Formel (I), in denen $R^1$ für die Reste der Formeln

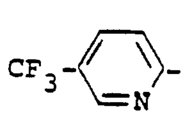

,

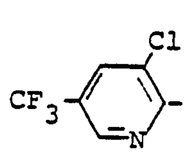

,

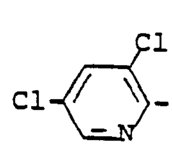

,

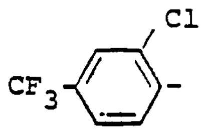 , 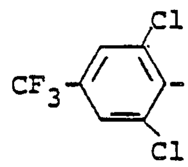 oder 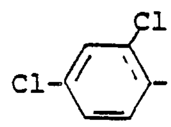

steht.

Verwendet man das rechtsdrehende Enantiomere des 2-[4-(5-Trifluormethyl-pyridyl-2-oxy)-phenoxy]-propionsäurechlorids und Ethylenglykol-mono-benzylether als Ausgangsstoffe, so kann der Verlauf des erfindungsgemäßen Verfahrens (a) durch das folgende Formelschema wiedergegeben werden :

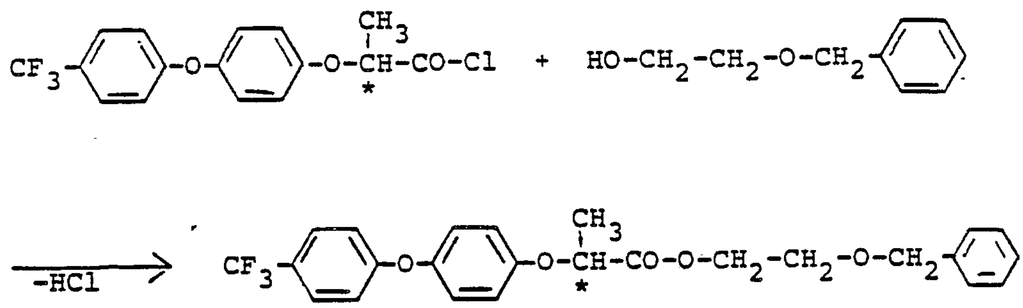


Verwendet man 4-(2-Chlor-4-trifluormethyl-phenoxy)-phenol und das linksdrehende Enantiomere des 2-Tosyloxy-propionsäure-(2-benzyloxy)-ethylesters als Ausgangsstoffe, so kann der Verlauf des erfindungsgemäßen Verfahrens (b) durch das folgende Formelschema wiedergegeben werden :

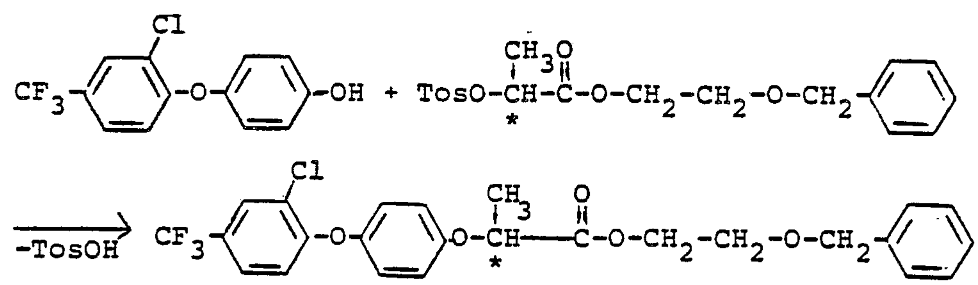


Tos = 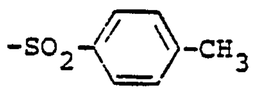 (=Tosyl)

Die bei dem erfindungsgemäßen Verfahren (a) als Ausgangsstoffe benötigten rechtsdrehenden Enantiomeren von Phenoxypropionsäure-chloriden sind durch die Formel (II) allgemein definiert. In dieser Formel steht $R^1$ vorzugsweise für diejenigen Reste, die bereits im Zusammenhang mit der Beschreibung der erfindungsgemäßen Stoffe vorzugsweise für den Substituenten $R^1$ genannt wurden.

Die rechtsdrehenden Enantiomeren der Phenoxypropionsäurechloride der Formel (II) sind bekannt oder lassen sich nach bekannten Methoden in einfacher Weise herstellen (vgl. DE-OS 2 758 002). So kann man die Stoffe der Formel (II) zum Beispiel dadurch erhalten, daß man die zugrundeliegenden Säuren mit Thionylchlorid umsetzt.

Die bei dem erfindungsgemäßen Verfahren (a) weiterhin als Ausgangsstoffe benötigten Hydroxyverbindungen sind durch die Formel (III) allgemein definiert. In dieser Formel haben $R^2$, $R^3$, $R^4$, Y und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Die Hydroxyverbindungen der Formel (III) sind bekannt oder lassen sich nach bekannten Methoden in einfacher Weise herstellen.

Die bei dem erfindungsgemäßen Verfahren (b) als Ausgangsstoffe benötigten Phenol-Derivate sind durch die Formel (IV) definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Phenol-Derivate der Formel (IV) sind bekannt oder lassen sich nach bekannten Methoden in

einfacher Weise herstellen (vgl. DE-OS 2 758 002, DE-OS 2 812 571, EP-OS 483 und EP-OS 1 473).

Die bei dem erfindungsgemäßen Verfahren (b) weiterhin als Ausgangssubstanzen benötigten linksdrehenden Enantiomeren der Propionsäure-Derivate sind durch die Formel (V) allgemein definiert. In dieser Formel haben $R^2$, $R^3$, $R^4$, Y und n vorzugsweise diejenigen Bedeutungen, die bereits im Zusammenhang mit der Beschreibung der erfindungsgemäßen Stoffe der Formel (I) vorzugsweise für diese Substituenten bzw. für diesen Index genannt wurden. Z steht für Tosyl

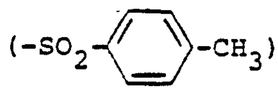 oder Mesyl $(-SO_2-CH_3)$

Die linksdrehenden Enantiomeren der Propionsäuren-Derivate der Formel (V) sind bisher noch nicht bekannt. Sie lassen sich jedoch in einfacher Weise herstellen, indem man Hydroxyverbindungen der Formel

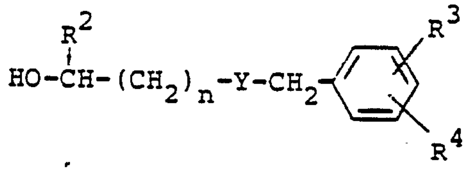 (III)

in welcher $R^2$, $R^3$, $R^4$, Y und n die oben angegebene Bedeutung haben, mit linksdrehenden Enantiomeren der Milchsäure-Derivate der Formel

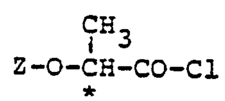 (VI)

in welcher Z die oben angegebene Bedeutung hat, gegebenenfalls in Gegenwart eines Säureakzeptors sowie gegebenenfalls in Gegenwart eines Verdünnungsmittels umsetzt.

Die bei der Herstellung der linksdrehenden Enantiomeren der Propionsäure-Derivate der Formel (V) als Ausgangsstoffe benötigten Hydroxyverbindungen der Formel (III) wurden bereits im Zusammenhang mit der Beschreibung des erfindungsgemäßen Verfahrens (a) behandelt. Die für die Umsetzung zur Synthese der Verbindungen der Formel (V) außerdem als Reaktionskomponenten benötigten linksdrehenden Enantiomeren der Milchsäure-Derivate der Formel (VI) sind bekannt oder lassen sich nach bekannten Methoden in einfacher Weise herstellen.

Die erfindungsgemäßen Verfahren (a) und (b) sowie auch das Verfahren zur Herstellung der Stoffe der Formel (V) werden vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutylketon, Ester, wie Essigsäure-methylester und -ethylester, Nitrile, wie z. B. Acetonitril und Propionitril, Amide, wie z. B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können sowohl bei den erfindungsgemäßen Verfahren (a) und (b) als auch bei dem Verfahren zur Herstellung der Verbindungen der Formel (V) alle üblicherweise für derartige Umsetzungen verwendbaren Säurebindemittel eingesetzt werden. Vorzugsweise in Frage kommen Alkalihydroxide, Erdalkalihydroxide und -oxide, wie z. B. Natrium- und Kaliumhydroxid, Calciumhydroxid und Calciumoxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzyl-amin, 1,5-Diazabicyclo [4,3,0] non-5-en (DBN), 1,8-Diazabicyclo [5,4,0] undec-7-en (DBU) und Pyridin.

Die Reaktionstemperaturen können sowohl bei den erfindungsgemäßen Verfahren (a) und (b) als auch bei dem Verfahren zur Herstellung der Verbindungen der Formel (V) innerhalb eines größeren Bereiches variiert werden. Im allgemeinen arbeitet man jeweils bei Temperaturen zwischen — 20 °C und + 160 °C, vorzugsweise zwischen — 10 °C und + 100 °C.

Die erfindungsgemäßen Verfahren (a) und (b) und auch das Verfahren zur Herstellung der Verbindungen der Formel (V) werden im allgemeinen unter Normaldruck durchgeführt. Es ist jedoch auch möglich, unter erhöhtem oder vermindertem Druck zu arbeiten.

Zur Herstellung derjenigen Verbindungen der Formel (I), in denen Y für $SO_m$ steht und m für 1 oder 2 steht, werden diejenigen Stoffe der Formel (I), in denen Y für Schwefel steht, nach üblichen Methoden mit

der jeweils benötigten Menge oder auch mit einem Überschuß an Oxidationsmittel, gegebenenfalls in Gegenwart eines Katalysators sowie in Gegenwart eines Verdünnungsmittels oxidiert.

Als Oxidationsmittel können dabei alle üblichen sauerstoffabgegebenden Oxidationsmittel eingesetzt werden. Vorzugsweise in Frage kommen Wasserstoffperoxid, Peressigsäure und m-Chlorbenzoesäure.

Als Verdünnungsmittel kommen bei der Durchführung dieser Oxidation alle üblicherweise für derartige Oxidationen verwendbaren organischen Lösungsmittel in Betracht. Vorzugsweise verwendbar sind Eisessig oder Methylenchlorid.

Als Katalysatoren können bei dieser Oxidation alle üblicherweise für derartige Oxidationen verwendbaren Reaktionsbeschleuniger eingesetzt werden. Vorzugsweise verwendbar sind Ameisensäure und Schwefelsäure.

Die Temperaturen können bei der Oxidation innerhalb eines bestimmten Bereiches variiert werden. Im allgemeinen arbeitet man zwischen — 20 °C und + 50 °C, vorzugsweise zwischen 0 °C und + 40 °C.

Bei der Durchführung der Oxidation setzt man die Ausgangsverbindung der Formel (I) im allgemeinen mit der jeweils berechneten Menge oder mit einem geringen Überschuß an Oxidationsmittel um. Die Aufarbeitung erfolgt jeweils nach üblichen Methoden.

Zur Durchführung der erfindungsgemäßen Verfahren (a) und (b) sowie des Verfahrens zur Herstellung der Verbindungen der Formel (V) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Verfahren. Im allgemeinen geht man so vor, daß man das Reaktionsgemisch mit Wasser versetzt, mehrfach mit einem mit Wasser wenig mischbaren organischen Lösungsmittel extrahiert, die vereinigten organischen Phasen trocknet und durch Abziehen des Lösungsmittels einengt.

Die neuen Verbindungen fallen zum Teil in Form von Ölen an, die sich zum Teil nicht unzersetzt destillieren lassen, jedoch durch sogenanntes « Andestillieren », d. h. durch längeres Erhitzen unter vermindertem Druck auf mäßig erhöhte Temperaturen von den letzten flüchtigen Anteilen befreit und auf diese Weise gereinigt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feintsverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als

Hilfslösungsmittel verwendet werden. Als flüssige Lösungsmittel kommen im wesentlichen in Frage : Aromaten, wie Xylol, Toluol, oder Alkylnaphthaline, chlorierte Aromaten und chlorierte aliphatische Kohlenwasserstoffe, wie Chlorbenzole, Chlorethylen oder Methylenchlorid, aliphatische Kohlenwasserstoffe, wie Cyclohexan oder Paraffine, z. B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage : z. B. Ammoniumsalze und natürliche Gesteinsmehle, wie Kaoline, Tonerden, Talkum, Kreide Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannten Herbizide wie z. B. 1-Amino-6-ethylthio-3-(2,2-dimethylpropyl)-1,3,5-triazin-2,4 (1H,3H)-dion oder N-(2-Benzthiazolyl)-N,N'-dimethyl-harnstoff zur Unkrautbekämpfung in Getreide ; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

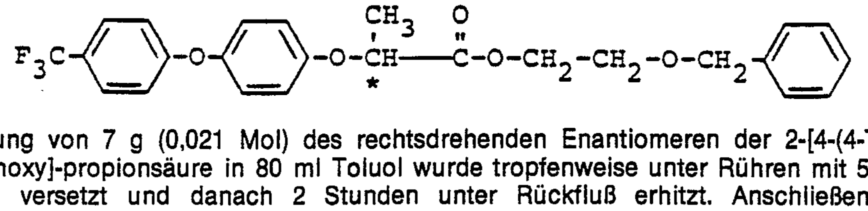

Eine Lösung von 7 g (0,021 Mol) des rechtsdrehenden Enantiomeren der 2-[4-(4-Trifluormethylphenoxy)-phenoxy]-propionsäure in 80 ml Toluol wurde tropfenweise unter Rühren mit 5 g (0,042 Mol) Thionylchlorid versetzt und danach 2 Stunden unter Rückfluß erhitzt. Anschließend wurde das Reaktionsgemisch durch Abziehen des Lösungsmittels eingeengt und bei 0 °C bis 5 °C unter Rühren in eine Lösung von 2,7 g (0,017 Mol) Glykol-mono-benzylether und 3 g (0,03 Mol) Triethylamin in 50 ml Toluol gegeben. Man rührte weitere 16 Stunden bei Raumtemperatur und arbeitete dann auf, indem man das Reaktionsgemisch mit Wasser versetzte, die vereinigten organischen Phasen trocknete und unter vermindertem Druck einengte. Der verbleibende Rückstand wurde durch leichtes Erwärmen im Hochvakuum von Resten an flüchtigen Anteilen befreit. Man erhielt auf diese Weise 6,2 g (62,6 % der Theorie) an rechtsdrehendem Enantiomeren des 2-[4-(4-Trifluormethyl-phenoxy)-phenoxy]-propionsäure-(2-benzyloxy)-ethylester.

Drehwert : $[\alpha]_D^{24} = +4{,}2°$
(1-molare Lösung in Chloroform ;
Küvettenlänge = 10 cm).

Beispiel 2

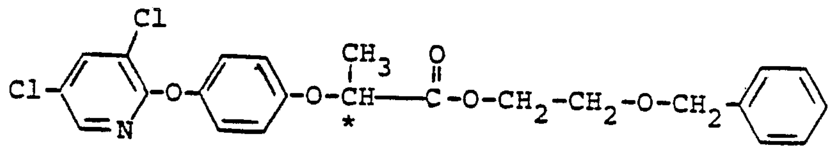

Ein Gemisch aus 25,6 g (0,1 Mol) 4-(3,5-Dichlor-pyridyl-2-oxy)-phenol, 37,8 g (0,1 Mol) an linksdrehendem Enantiomeren des 2-Tosyloxy-propionsäure-(2-benzyloxy)-ethylesters und 16,6 g (0,12 Mol) Kaliumcarbonat in 200 ml Acetonitril wurde 14 Stunden unter Rückfluß erhitzt. Danach wurde das Reaktionsgemisch auf Raumtemperatur abgekühlt und mit 400 ml Wasser versetzt. Es wurde zweimal mit je 200 ml Toluol extrahiert. Die vereinigten organischen Phasen wurden über Natriumsulfat getrocknet und durch Abziehen des Lösungsmittels unter vermindertem Druck eingeengt. Der verbleibende Rückstand wurde durch leichtes Erwärmen im Hochvakuum von Resten an flüchtigen Anteilen befreit. Man erhielt auf diese Weise 28 g (60 % der Theorie) an rechtsdrehendem Enantiomeren des 2-[4-(3,5-Dichlorpyridyl-2-oxy)-phenoxy]-propionsäure-(2-benzyloxy)-ethylesters.

Drehwert : $[\alpha]_D^{24} = +10{,}2°$
(1-molare Lösung in Chloroform ;
Küvettenlänge = 10 cm).

Herstellung des Ausgangsproduktes

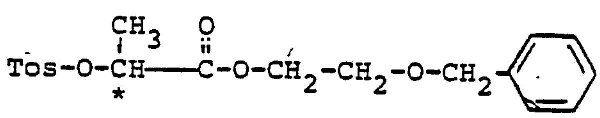

(V-1)

5.25 g (0,02 Mol) des linksdrehenden Enantiomeren des Milchsäurechlorid-tosylats wurden bei 20 °C unter Rühren in ein Gemisch aus 3,32 g (0,02 Mol) Glykol-mono-benzyl-ether, 2 g (0,02 Mol) Triethylamin und 50 ml Toluol gegeben. Man rührte weitere 14 Stunden bei 70 °C und arbeitete dann auf, indem man das Reaktionsgemisch mit 100 ml Wasser versetzte, dann mehrfach mit Toluol extrahierte, die vereinigten organischen Phasen trocknete und durch Abziehen des Lösungsmittels unter vermindertem Druck einengte. Man erhielt auf diese Weise 6,3 g (80,5 % der Theorie) an linksdrehendem Enantiomeren des 2-Tosyloxy-propion-säure-(2-benzyloxy)-ethylesters.

Drehwert : $[\alpha]_D^{24} = -10{,}2°$
(1-molare Lösung in Chloroform ;
Küvettenlänge = 10 cm).

Nach den in den Beispielen 1 und 2 angegebenen Methoden wurden auch die in der folgenden Tabelle formelmäßig aufgeführten Stoffe der Formel (I) hergestellt.

(Siehe Tabelle 1 Seite 9 f.)

Tabelle 1

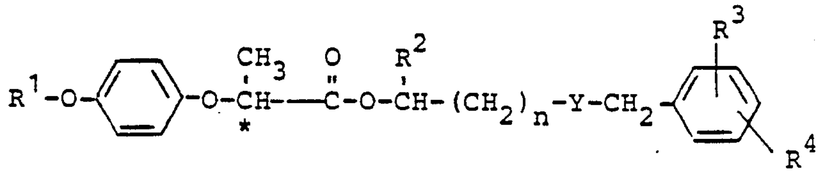

(I)

| Beispiel Nr. | $R^1$ | $R^2$ | n | Y | $R^3$ | $R^4$ | Drehwert $[\alpha]_D^{24}$ |
|---|---|---|---|---|---|---|---|
| 3 | 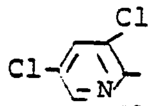 | H | 1 | 0 | 2-F | H | + 7,9 |
| 4 | 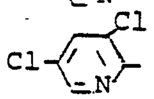 | H | 1 | 0 | 4-Cl | H | + 8,7 |
| 5 | 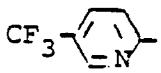 | H | 1 | 0 | H | H | + 10,0 |
| 6 | 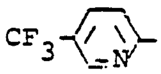 | H | 1 | 0 | 2-F | H | + 9,1 |
| 7 | 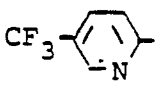 | H | 1 | 0 | 4-Cl | H | + 9,9 |
| 8 | 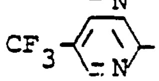 | H | 2 | 0 | H | H | + 10,3 |
| 9 | 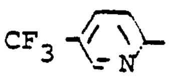 | H | 1 | S | H | H | + 14,1 |
| 10 | 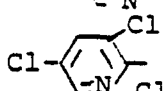 | $CH_3$ | 1 | 0 | H | H | + 3,1 |
| 11 | 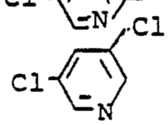 | H | 1 | S | H | H | + 10,5 |

Nach der im Beispiel 2 angegebenen Methode zur Herstellung der Verbindung (V-1) wurden auch die in der folgenden Tabelle formelmäßig aufgeführten Stoffe der Formel (V) synthetisiert.

Tabelle 2

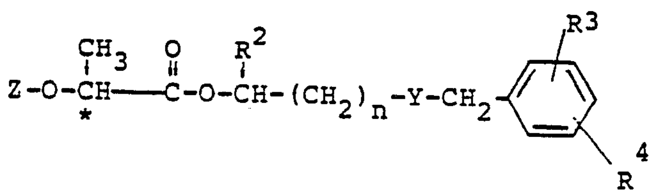

(V)

| Beispiel Nr. | Z | $R^2$ | n | Y | $R^3$ | $R^4$ | Drehwert $[\alpha]_D^{24}$ |
|---|---|---|---|---|---|---|---|
| (V-2) | Tos | H | 1 | 0 | 2-F | H | - 9,7 |
| (V-3) | Tos | H | 2 | 0 | H | H | - 10,3 |
| (V-4) | Tos | $CH_3$ | 1 | 0 | H | H | - 4,1 |
| (V-5) | Tos | H | 1 | 0 | 4-Cl | H | - 7,6 |
| (V-6) | Tos | H | 1 | S | H | H | - 10,9 |

Beispiel 12

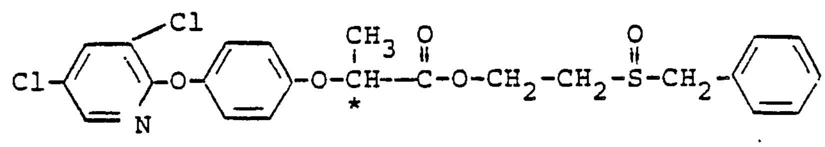

Zu einer Lösung von 24 g (0,05 Mol) des rechtsdrehenden Enantiomeren des 2-[4-(3,5-Dichlor-pyridyl-2-oxy)-phenoxy]-propionsäure-(2-benzylthio)-ethylesters in 100 ml Eisessig wurden bei +10 °C unter Rühren 5,7 g 30-prozentige wäßrige Wasserstoffperoxid-Lösung zugetropft. Das Reaktionsgemisch wurde 10 Stunden bei 20 °C gerührt und dann nacheinander mit 10 ml wäßriger Kaliumhydrogensulfit-Lösung und mit 200 ml Wasser versetzt. Zur weiteren Aufarbeitung wurde das Reaktionsgemisch zweimal mit je 100 ml Methylenchlorid extrahiert. Die vereinigten organischen Phasen wurden mit 50 ml gesättigter wäßriger Kaliumcarbonat-Lösung und dann zweimal mit je 100 ml Wasser gewaschen. Nach dem Trocknen und Abziehen des Lösungsmittels unter vermindertem Druck erhielt man 19 g (76 % der Theorie) an dem rechtsdrehenden Enantiomeren des 2-[4-(3,5-Dichlor-pyridyl-2-oxy)-phenoxy]-propionsäure-(2-benzylsulfinyl)-ethylesters in Form farbloser Kristalle vom Schmelzpunkt 112 °C.

Drehwert : $[\alpha]_D^{24} = + 12,2°$.

Beispiel 13

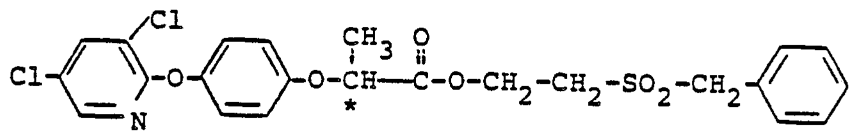

Zu einer Lösung von 24 g (0,05 Mol) des rechtsdrehenden Enantiomeren des 2-[4-(3,5-Dichlor-pyridyl-2-oxy)-phenoxy]-propionsäure-(2-benzylthio)-ethylesters in 150 ml Methylenchlorid werden bei 20 °C unter Rühren zunächst 5 g (0,11 Mol) Ameisensäure, dann bei 20 °C 0,5 ml Schwefelsäure und anschließend bei 10-20 °C 16,5 g 30 %ige wäßrige Wasserstoffperoxid-Lösung zugetropft. Das Reaktionsgemisch wurde 10 Stunden bei 20 °C gerührt und dann nacheinander mit 10 ml wäßriger Kaliumhydrogensulfit-Lösung und mit 200 ml Wasser versetzt. Zur weiteren Aufarbeitung wurde das Reaktionsgemisch zweimal mit je 100 ml Methylenchlorid extrahiert. Die vereinigten organischen Phasen wurden mit 50 ml gesättigter wäßriger Kaliumcarbonat-Lösung und dann zweimal mit je 100 ml Wasser gewaschen. Nach dem Trocknen und Abziehen des Lösungsmittels unter vermindertem Druck erhielt man 21 g (81 % der Theorie) an dem rechtsdrehenden Enantiomeren des 2-[4-(3,5-Dichlor-pyridyl-2oxy)-phenoxy]-propionsäure-(2-benzylsulfonyl)-ethylesters in Form eines gelben Öles vom Brechungsindex $n_D^{20} = 1,580\,7$.

Drehwert : $[\alpha]_D^{24} = + 15,1°$.

Beispiel A

Pre-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

Die erfindungsgemäßen Wirkstoffe zeigen in diesem Test eine sehr gute Wirksamkeit.

Beispiel B

Post-emergence-Test

Lösungsmittel : 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2 000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten :

0 % = keine Wirkung (wie unbehandelte Kontrolle)
100 % = totale Vernichtung

Die erfindungsgemäßen Wirkstoffe 2, 4, 5 und 7-9 zeigen in diesem Test eine sehr gute Wirksamkeit.

Tabelle
Post-emergence-Test
Gewächshaus

| Wirkstoff | Wirkstoffmenge kg/ha | % Schäden bzw. % Wirkung | | | |
|---|---|---|---|---|---|
| | | Zuckerrüben | Sojabohnen | Echinochloa | Setaria |
| (2) | 0,25 | 0 | 0 | 100 | 100 |
| (4) | 0,25 | 0 | 0 | 100 | 100 |
| (5) | 0,25 | 0 | 0 | 100 | 95 |
| (7) | 0,25 | 0 | 0 | 100 | 99 |
| (8) | 0,25 | 0 | 0 | 100 | 99 |
| (9) | 0,25 | 0 | 25 | 99 | 99 |

**Patentansprüche**

1. Rechtsdrehende Enantiomere der Phenoxypropionsäure-Derivate der Formel

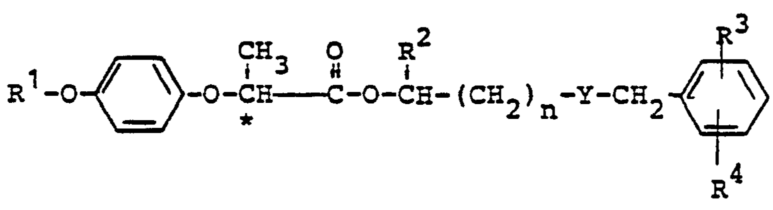

(I)

in welcher $R^1$ für die Reste der Formeln

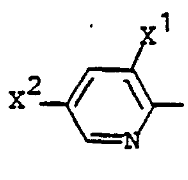

oder

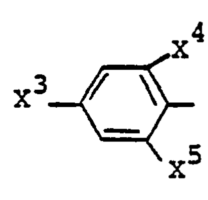

steht, worin

$X^1$ für Wasserstoff oder Halogen steht,

$X^2$ für Halogen oder Trifluormethyl steht,
$X^3$ für Halogen oder Trifluormethyl steht,
$X^4$ für Wasserstoff oder Halogen steht und
$X^5$ für Wasserstoff oder Halogen steht,
Y für Sauerstoff oder den Rest $SO_m$ steht, wobei
m für 0, 1 oder 2 steht,
$R^2$ für Wasserstoff oder Methyl steht,
n für 1 oder 2 steht und
$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen.

2. Verfahren zur Herstellung der rechtsdrehenden Enantiomeren der Phenoxypropionsäure-Derivate der Formel

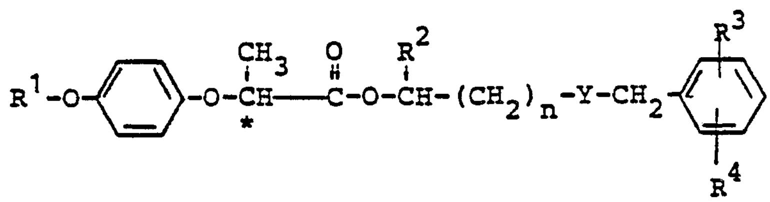

(I)

in welcher $R^1$ für die Reste der Formeln

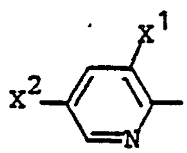

oder

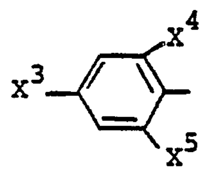

steht, worin
$X^1$ für Wasserstoff oder Halogen steht,
$X^2$ für Halogen oder Trifluormethyl steht,
$X^3$ für Halogen oder Trifluormethyl steht,
$X^4$ für Wasserstoff oder Halogen steht und
$X^5$ für Wasserstoff oder Halogen steht,
Y für Sauerstoff oder den Rest $SO_m$ steht, wobei
m für 0, 1 oder 2 steht,
$R^2$ für Wasserstoff oder Methyl steht,
n für 1 oder 2 steht und
$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe stehen,
dadurch gekennzeichnet, daß man

a) rechtsdrehende Enantiomere von Phenoxypropionsäurechloriden der Formel

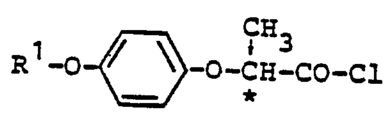

(II)

in welcher $R^1$ die oben angegebene Bedeutung hat, mit Hydroxyverbindungen der Formel

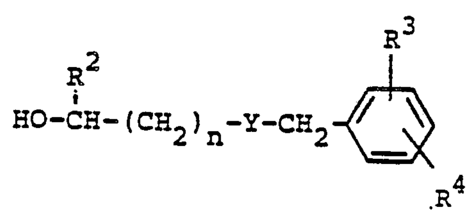

(III)

in welcher $R^2$, $R^3$, $R^4$, Y und n die oben angegebene Bedeutung haben, gegebenenfalls in Gegenwart eines Säureakzeptors sowie gegebenenfalls in Gegenwart eines Verdünnungsmittels umsetzt und gegebenenfalls anschließend oxidiert,
oder

b) Phenol-Derivate der Formel

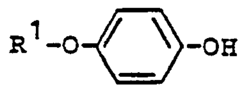

(IV)

in welcher $R^1$ die oben angegebene Bedeutung hat, mit linksdrehenden Enantiomeren von Propionsäure-Derivaten der Formel

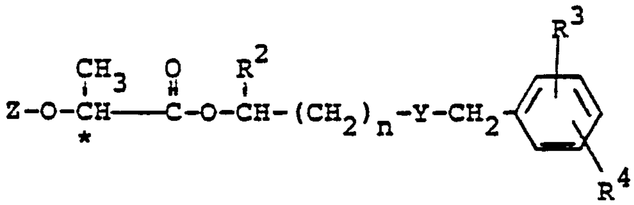

(V)

in welcher

$R^2$, $R^3$, $R^4$, Y und n die oben angegebene Bedeutung haben und

Z für Tosyl oder Mesyl steht,

gegebenenfalls in Gegenwart eines Säureakzeptors sowie gegebenenfalls in Gegenwart eines Verdünnungsmittels umsetzt und gegebenenfalls anschließend oxidiert.

3. Herbizide Mittel, gekennzeichnet durch einen Gehalt an mindestens einem rechtsdrehenden Enantiomeren eines Phenoxypropionsäure-Derivats der Formel (I).

4. Verwendung von rechtsdrehenden Enantiomeren der Phenoxypropionsäure-Derivate der Formel (I) als Herbizide.

5. Verfahren zur Bekämpfung von Unkräutern, dadurch gekennzeichnet, daß man rechtsdrehende Enantiomere der Phenoxypropionsäure-Derivate der Formel (I) auf die Unkräuter und/oder deren Lebensraum ausbringt.

6. Verfahren zur Herstellung von herbiziden Mitteln, dadurch gekennzeichnet, daß man rechtsdrehende Enantiomere der Phenoxypropionsäure-Derivate der Formel (I) mit Streckmitteln und/oder oberflächenaktiven Stoffen vermischt.

7. Linksdrehende Enantiomere der Propionsäure-Derivate der Formel

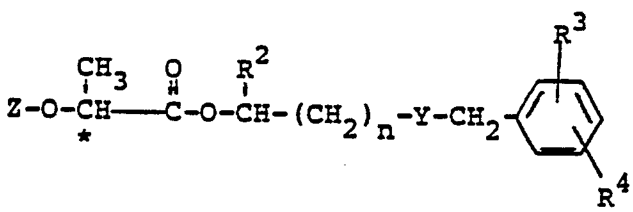

(V)

in welcher

Z für Tosyl oder Mesyl steht,

Y für Sauerstoff oder dem Rest $SO_m$ steht, wobei

m für 0, 1 oder 2 steht,

$R^2$ für Wasserstoff oder Methyl steht,

n für 1 oder 2 steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder Alkoxycarbonyl mit 1 bis 4 Kohlenstoffatomen in der Alkoxygruppe steht.

8. Verfahren zur Herstellung von linksdrehenden Enantiomeren der Propionsäure-Derivate der Formel

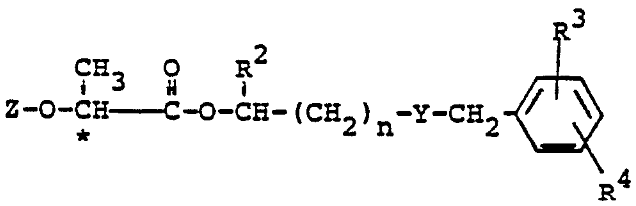

(V)

in welcher

Z für Tosyl oder Mesyl steht,

Y für Sauerstoff oder den Rest $SO_m$ steht, wobei

m für 0, 1 oder 2 steht,

$R^2$ für Wasserstoff oder Methyl steht,

n für 1 oder 2 steht und

$R^3$ und $R^4$ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Nitro, Cyano oder

Alkoxycarbonyl mit 1 bis 4 Kohlenstoffatomen in der Alkoxygruppe stehen, dadurch gekennzeichnet, daß man Hydroxyverbindungen der Formel

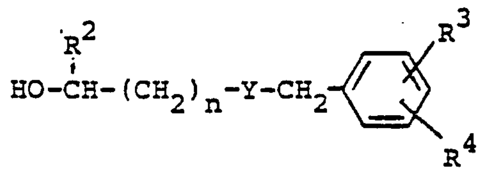

(III)

in welcher $R^2$, $R^3$, $R^4$, Y und n die oben angegebene Bedeutung haben, mit linksdrehenden Enantiomeren der Milchsäure-Derivate der Formel

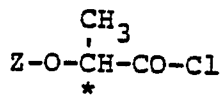

(VI)

in welcher Z die oben angegebene Bedeutung hat, gegebenenfalls in Gegenwart eines Säureakzeptors sowie gegebenenfalls in Gegenwart eines Verdünnungsmittels umsetzt.

**Claims**

1. Dextrorotatory enantiomers of phenoxypropionic acid derivatives of the formula

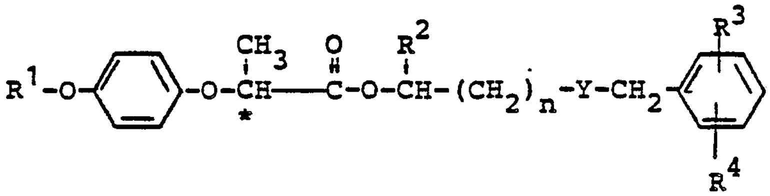

(I)

in which $R^1$ represents the radicals of the formulae

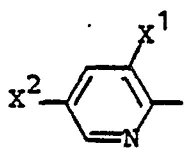

or

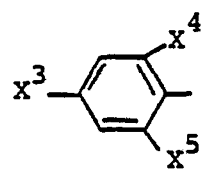

wherein

$X^1$ represents hydrogen or halogen,
$X^2$ represents halogen or trifluoromethyl,
$X^3$ represents halogen or trifluoromethyl,
$X^4$ represents hydrogen or halogen and
$X^5$ represents hydrogen or halogen,
Y represents oxygen or the radical $SO_m$, wherein
m represents 0, 1 or 2,
$R^2$ represents hydrogen or methyl,
n represents 1 or 2 and
$R^3$ and $R^4$ independently of one another represent hydrogen, halogen, alkyl with 1 to 4 carbon atoms, alkoxy with 1 to 4 carbon atoms, alkylthio with 1 to 4 carbon atoms, nitro, cyano or alkoxycarbonyl with 1 to 4 carbon atoms in the alkoxy group.

2. Process for the preparation of the dextrorotatory enantiomers of the phenoxypropionic acid derivatives of the formula

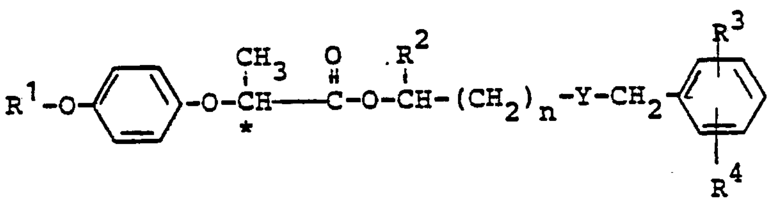

(I)

in which $R^1$ represents the radicals of the formulae

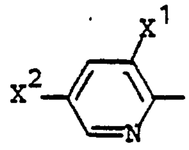

or

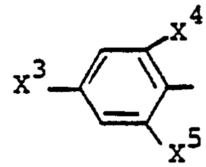

wherein
$X^1$ represents hydrogen or halogen,
$X^2$ represents halogen or trifluoromethyl,
$X^3$ represents halogen or trifluoromethyl,
$X^4$ represents hydrogen or halogen and
$X^5$ represents hydrogen or halogen,
Y represents oxygen or the radical $SO_m$, wherein
m represents 0, 1 or 2,
$R^2$ represents hydrogen or methyl,
n represents 1 or 2 and
$R^3$ and $R^4$ independently of one another represent hydrogen, halogen, alkyl with 1 to 4 carbon atoms, alkoxy with 1 to 4 carbon atoms, alkylthio with 1 to 4 carbon atoms, nitro, cyano or alkoxycarbonyl with 1 to 4 carbon atoms in the alkoxy group,
characterised in that

a) dextrorotatory enantiomers of phenoxypropionyl chlorides of the formula

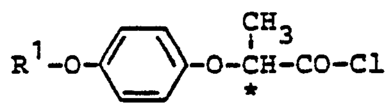

(II)

in which $R^1$ has the abovementioned meaning, are reacted with hydroxy compounds of the formula

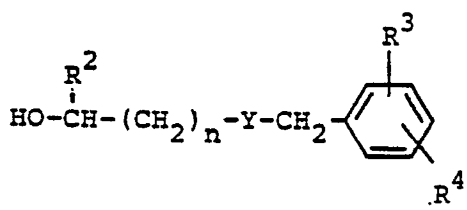

(III)

in which $R^2$, $R^3$, $R^4$, Y and n have the abovementioned meaning, if appropriate in the presence of an acid acceptor and if appropriate in the presence of a diluent, and, if appropriate, the product is then oxidised, or

b) phenol derivates of the formula

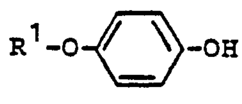

(IV)

in which $R^1$ has the abovementioned meaning, are reacted with laevorotatory enantiomers of propionic acid derivatives of the formula

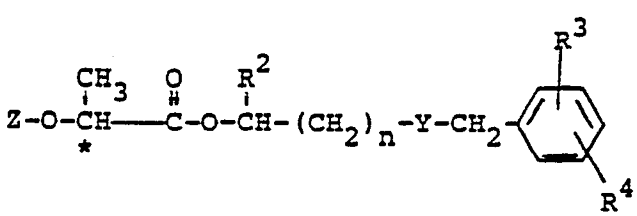

(V)

in which
$R^2$, $R^3$, $R^4$, Y and n have the abovementioned meaning and
Z represents tosyl or mesyl,
if appropriate in the presence of an acid acceptor and if appropriate in the presence of a diluent, and, if appropriate, the product in then oxidised.

3. Herbicidal agents, characterised in that they contain at least one dextrorotatory enantiomer of a phenoxypropionic acid derivative of the formula (I).

4. Use of dextrorotatory enantiomers of phenoxypropionic acid derivatives of the formula (I) as herbicides.

5. Method of combating weeds, characterised in that dextrorotatory enantiomers of phenoxypropionic acid derivatives of the formula (I) are applied to the weeds and/or their environment.

6. Process for the preparation of herbicidal agents, characterised in that dextrorotatory enantiomers of phenoxypropionic acid derivatives of the formula (I) are mixed with extenders and/or surface-active substances.

7. Laevorotatory enantiomers of propionic acid derivatives of the formula

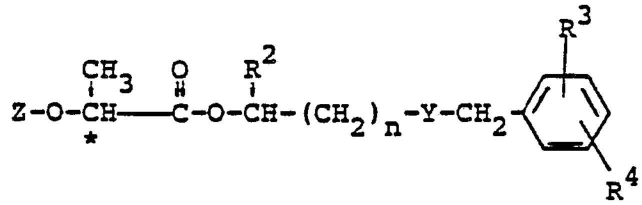

(V)

in which

Z represents tosyl or mesyl,

Y represents oxygen or the radical $SO_m$,

wherein

m represents 0, 1 or 2,

$R^2$ represents hydrogen or methyl,

n represents 1 or 2 and

$R^3$ and $R^4$ independently of one another represent hydrogen, halogen, alkyl with 1 to 4 carbon atoms, alkoxy with 1 to 4 carbon atoms, alkylthio with 1 to 4 carbon atoms, nitro, cyano or alkoxycarbonyl with 1 to 4 carbon atoms in the alkoxy group.

8. Process for the preparation of laevorotatory enantiomers of propionic acid derivatives of the formula

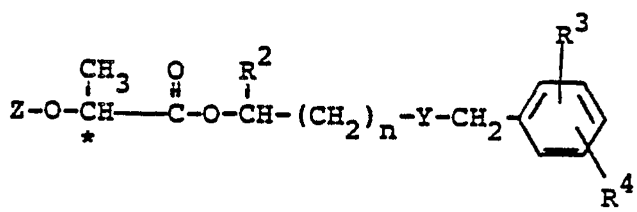

(V)

in which

Z represents tosyl or mesyl,

Y represents oxygen or the radical $SO_m$, wherein

m represents 0, 1 or 2,

$R^2$ represents hydrogen or methyl,

n represents 1 or 2 and

$R^3$ and $R^4$ independently of one another represent hydrogen, halogen, alkyl with 1 to 4 carbon atoms, alkoxy with 1 to 4 carbon atoms, alkylthio with 1 to 4 carbon atoms, nitro, cyano or alkoxycarbonyl with 1 to 4 carbon atoms in the alkoxy group,

characterised in that hydroxy compounds of the formula

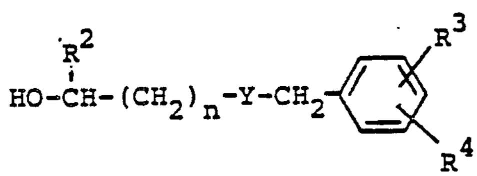

(III)

in which $R^2$, $R^3$, $R^4$, Y and n have the abovementioned meaning, are reacted with laevorotatory enantiomers of lactic acid derivatives of the formula

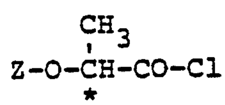

(VI)

in which Z has the abovementioned meaning, if appropriate in the presence of an acid acceptor and if appropriate in the presence of a diluent.

**Revendications**

1. Enantiomères dextrogyres des dérivés de l'acide phénoxypropionique répondant à la formule

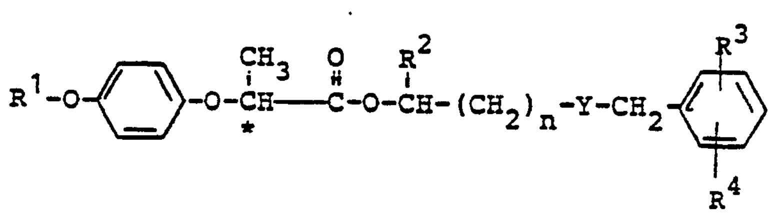 (I)

dans laquelle $R^1$ représente les groupes de formules

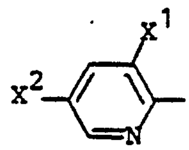 ou 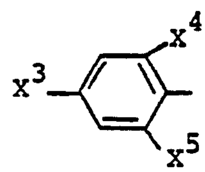

dans lesquelles
$X^1$ représente l'hydrogène ou un halogène,
$X^2$ représente un halogène ou un groupe trifluorométhyle,
$X^3$ représente un halogène ou un groupe trifluorométhyle,
$X^4$ représente l'hydrogène ou un halogène, et
$X^5$ représente l'hydrogène ou un halogène,
Y représente l'oxygène ou le groupe $SO_m$ dans lequel
m est égal à 0, 1 ou 2,
$R^2$ représente l'hydrogène ou un groupe méthyle,
n est égal à 1 ou 2, et
$R^3$ et $R^4$ représentent chacun, indépendamment l'un de l'autre, l'hydrogène, un halogène, un groupe alkyle en $C_1$-$C_4$, alcoxy en $C_1$-$C_4$, alkylthio en $C_1$-$C_4$, nitro, cyano ou alcoxycarbonyle contenant 1 à 4 atomes de carbone dans la partie alcoxy.

2. Procédé de préparation des énantiomères dextrogyres des dérivés de l'acide phénoxypropionique de formule

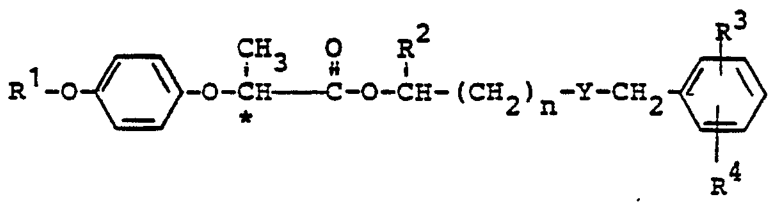 (I)

dans laquelle $R^1$ représente les groupes de formules

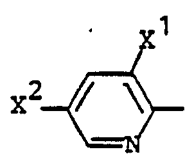 ou 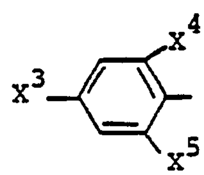

dans lesquelles
$X^1$ représente l'hydrogène ou un halogène,
$X^2$ représente un halogène ou un groupe trifluorométhyle,
$X^3$ représente un halogène ou un groupe trifluorométhyle,

$X^4$ représente l'hydrogène ou un halogène, et
$X^5$ représente l'hydrogène ou un halogène,
Y représente l'oxygène ou le groupe $SO_m$ dans lequel
m est égal à 0, 1 ou 2,
$R^2$ représente l'hydrogène ou un groupe méthyle,
n est égal à 1 ou 2, et
$R^3$ et $R^4$ représente chacun, indépendamment l'un de l'autre, l'hydrogène, un halogène, un groupe alkyle en $C_1$-$C_4$, alcoxy en $C_1$-$C_4$, alkylthio en $C_1$-$C_4$, nitro, cyano ou alcoxycarbonyle contenant 1 à 4 atomes de carbone dans la partie alcoxy,

caractérisé en ce que :

a) on fait réagir les énantiomères dextrogyres de chlorures d'acides phénoxy-propioniques de formule

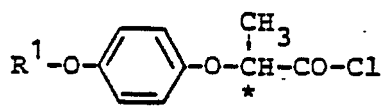

(II)

dans laquelle $R^1$ a les significations indiquées ci-dessus, avec des composés hydroxylés de formule

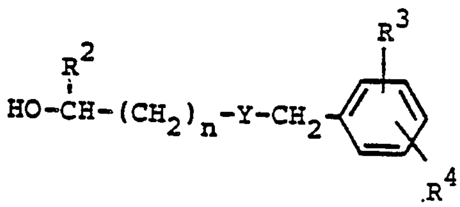

(III)

dans laquelle $R^2$, $R^3$, $R^4$, Y et n ont les significations indiquées ci-dessus, éventuellement en présence d'un accepteur d'acide et éventuellement en présence d'un diluant, et le cas échéant, on soumet ensuite à oxydation,

ou bien

b) on fait réagir des dérivés du phénol de formule

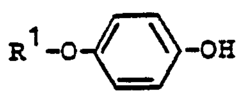

(IV)

dans laquelle $R^1$ a les significations indiquées ci-dessus, avec les énantiomères lévogyres de dérivés de l'acide propionique répondant à la formule

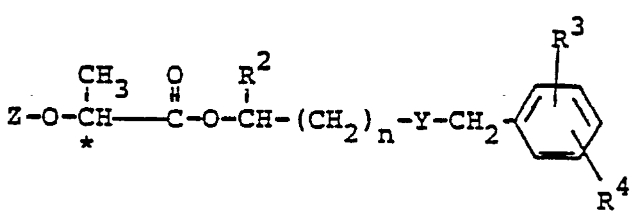

(V)

dans laquelle $R^2$, $R^3$, $R^4$, Y et n ont les significations indiquées ci-dessus, et Z représente un groupe toluène-sulfonyle ou méthane-sulfonyle, éventuellement en présence d'un accepteur d'acide et éventuellement en présence d'un diluant, et le cas échéant on soumet ensuite à oxydation.

3. Produits herbicides caractérisés en ce qu'ils contiennent au moins un énantiomère dextrogyre d'un dérivé de l'acide phénoxypropionique de formule I.

4. Utilisation des énantiomères dextrogyres des dérivés de l'acide phénoxypropionique de formule I en tant qu'herbicides.

5. Procédé pour combattre les mauvaises herbes, caractérisé en ce que l'on applique sur les mauvaises herbes et/ou leur habitat des énantiomères dextrogyres des dérivés de l'acide phénoxypropionique répondant à la formule I.

6. Procédé de préparation de produits herbicides, caractérisé en ce que l'on mélange des énantiomères dextrogyres de dérivés de l'acide phénoxypropionique répondant à la formule I avec des diluants et/ou des agents tensioactifs.

7. Enantiomères lévogyres de dérivés de l'acide propionique répondant à la formule

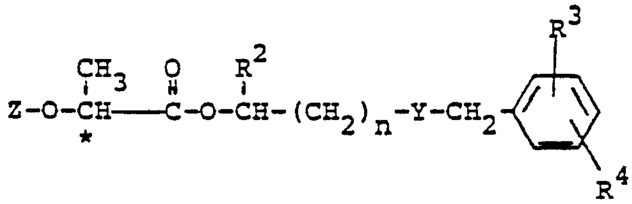 (V)

dans laquelle
Z représente un groupe toluène-sulfonyle ou méthane-sulfonyle,
Y représente l'oxygène ou le groupe $SO_m$ dans lequel
m est égal à 0, 1 ou 2,
$R^2$ représente l'hydrogène ou un groupe méthyle,
n est égal à 1 ou 2, et
$R^3$ et $R^4$ représentent chacun, indépendamment l'un de l'autre, l'hydrogène, un halogène, un groupe alkyle en $C_1$-$C_4$, alcoxy en $C_1$-$C_4$, alkylthio en $C_1$-$C_4$, nitro, cyano ou alcoxycarbonyle contenant 1 à 4 atomes de carbone dans la partie alcoxy.

8. Procédé de préparation des énantiomères lévogyres de dérivés de l'acide propionique de formule

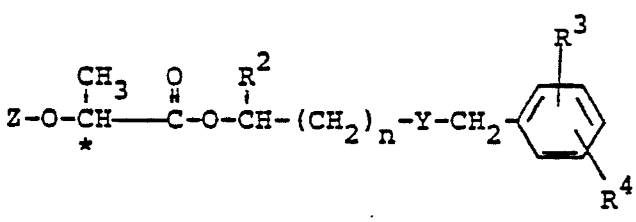 (V)

dans laquelle
Z représente un groupe toluène-sulfonyle ou méthane-sulfonyle,
Y représente l'oxygène ou le groupe $SO_m$ dans lequel
m est égal à 0, 1 ou 2,
$R^2$ représente l'hydrogène ou un groupe méthyle,
n est égal à 1 ou 2, et
$R^3$ et $R^4$ représentent chacun, indépendamment l'un de l'autre, l'hydrogène, un halogène, un groupe alkyle en $C_1$-$C_4$, alcoxy en $C_1$-$C_4$, alkylthio en $C_1$-$C_4$, nitro, cyano ou alcoxycarbonyle contenant 1 à 4 atomes de carbone dans la partie alcoxy,
caractérisé en ce que on fait réagir des composés hydroxylés de formule

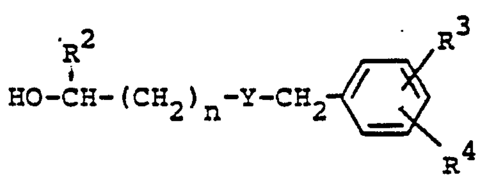 (III)

dans laquelle $R^2$, $R^3$, $R^4$, Y et n ont les significations indiquées ci-dessus, avec les énantiomères lévogyres des dérivés de l'acide lactique de formule

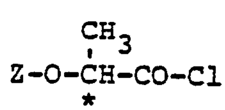 (VI)

dans laquelle Z a les significations indiquées ci-dessus, éventuellement en présence d'un accepteur d'acide et éventuellement en présence d'un diluant.